# EUROPEAN PATENT SPECIFICATION

(11) **EP 0 469 773 B1**
(45) Date of publication and mention of the grant of the patent: **25.09.1996**
(21) Application number: 91306684.1
(22) Date of filing: 23.07.1991
(51) Int. Cl.: G01N 17/00, G01N 33/18, G01N 21/78

(54) **Iron (II) concentration monitoring for determining corrosion in boiler systems**
Überwachung des Eisen(II)gehalts zur Korrosionsbestimmung in Kesseleinrichtungen
Surveillence de la teneur en fer ferreux pour déterminer la corrosion dans des systèmes de chaudières

(30) Priority: 31.07.1990 US 560687
(43) Date of publication of application: 05.02.1992
(73) Proprietor: NALCO CHEMICAL COMPANY, Naperville Illinois 60563-1198 (US)
(72) Inventor: Pierce, Claudia C., Lisle, Illinois 60532 (US); Fowee, Roger W., Wheaton, Illinois 60187 (US); Banks, Rodney H., Naperville, Illinois 60563 (US)
(74) Representative: Harrison, David Christopher

(56) References cited:
- EP-A- 0 469 772
- US-A- 3 770 735
- US-A- 3 836 331
- WORLD PATENTS INDEX LATEST Week 8336, Derwent Publications Ltd., London, GB; AN 83-755598 & JP-A-58 127 152

## Description

Potential corrosion problems in boiler systems have presented the boiler operator with many unanswered questions over the past histories of operating these boiler systems. These problems have increased as the boiler systems themselves have become more sophisticated. Boiler systems that are operating at extremely high pressures and temperatures particularly require pretreatment of waters being used to feed the boilers to eliminate such contaminants as calcium and magnesium hardness, other inorganic salts such as ferrous and ferric salts, silicates, and the like.

Corrosion reactions in all of these boiler systems have been demonstrated by many previous researchers to be affected by many parameters such as oxygen content, water pH, temperature, pressure, water flow velocity, the formation of certain types of salts and scales, for example hardness scales, iron scales, particularly magnetite, and treatment with boiler water treatment chemicals which are designed to protect the boiler from corrosion, hardness scale deposits, oxygen induced corrosion, pH excursions, and the like.

Recently, it has been determined that ferrometals making up the boiler system units, which ferrometals can intrude a number of different types of iron containing steels, iron alloys, and the like, are faced with particularly difficult detection of corrosion problems from the point of view of determining when these boiler system units are experiencing corrosion and determining the reasons for the corrosion occurring.

As a result, our invention is related to the detection and determination of corrosion and of corrosion rates of boiler system ferrometals in contact with boiler waters, particularly those boiler waters containing boiler treatment chemicals purposely added to boiler waters, and boiler feed waters.

The chemistry of iron and its corrosion products in boiler systems has been summarized by W.E. Bornak in an article published In Corrosion (NACE), Volume 44, No. 3, March, 1988. Bornak describes measurement of different types of corrosion of ferrometals in contact with waters internal to boilers, particularly reviewing investigations of magnetite formation and corrosion related thereto in boilers. Bornak's article primarily summarizes the difficulties corrosion causes and presents several mechanisms to explain why corrosion is occurring. For example, he speaks of oxygen corrosion, the formation of magnetite and its protective characteristics when formed on a ferrometal surface in contact with boiler waters, and the breakdown of this magnetite layer under certain circumstances.

Bornak also talks generally In terms of the difficulties encountered by the production of hydrogen which is a byproduct of corrosion and which has been traditionally used to measure corrosion rates occurring in a operating boiler. According to Bornak, hydrogen atoms produced through corrosion or through some other side mechanism, can diffuse into the metal and be lost either by direct diffusion or by reactions with various forms of metal oxides encountered by the hydrogen Hydrogen lost in any of these ways is therefore not available for measurement and the detection based on hydrogen of actual corrosion in an operating boiler is therefore limited.

In the test published by Wiley Interscience Publications, John Wiley and Sons, entilled "Corrosion and Corrosion Control, An Introduction To Corrosion Science and Engineering", Third Edition, edited by H.H. Uhlig and R.W. Revie, basic electrochemical theory of corrosion is described in general for iron and steel corrosion in the presence of aqueous media. In anaerobic solutions, the cathodic reaction is presented as the formation of hydrogen. Since well operated boilers are operated in essentially oxygen free environments, thereby qualifying as anaerobic solutions, the cathodic reaction is primarily that reaction producing hydrogen However, because of the difficulties mentioned above in measuring hydrogen, particularly accurately measuring hydrogen in an operating boiler in the field, other techniques for accurately measuring real time corrosion phenomena are required.

In an article entitled "Dissolved Hydrogen Analyzer - A Tool for Boiler Corrosion Studies", by Jacklin and Wiltsey, published In Materials Protection and Performance, May 1971, the authors explain attempts to provide for a dissolved hydrogen analyzer which for the most part, though improved from these disclosures, is essentially the best analysis existing today for monitoring corrosion in an operating boiler system However, again because of the difficulties earlier mentioned, dissolved hydrogen is not a preferred method, although it has been the workhorse of the industry over the years.

Another article entitled "Hydrogen Analysis as a Method of Corrosion Monitoring in Boilers", by Joneson, published in Combustion, August, 1979, points out some of the difficulty in interpreting dissolved hydrogen data and distinguishing from hydrogen formed by generalized and localized corrosion reactions and hydrogen, for example, which might be generated from chemical decompositions of boiler water treatment chemicals or from some unknown interference.

Also in Corrosion, April 1984, Paper No. 57 entitled "Application of Dissolved Hydrogen Measurement Technique for Monitoring of Corrosion in High Pressure Boilers", by Mayer, et al, the authors state that detection and recognition of conditions leading to accelerated corrosion are essential in reducing short and long term metal loss. The authors emphasize hydrogen measurements be used frequently, but state that the results of the hydrogen measurements would be no more than a summary indication of many processes which may take place within the system and suggest combination of hydrogen tests with other analytical and operational parameters. However their techniques are primarily in monitoring hydrogen values as well as monitoring operational controls in an operating boiler.

In Corrosion, 1990, Paper No. 184 presented April 23-27, 1990 by Bane, et al, the authors speak again of internal boiler corrosion leading to the formation of magnetite and hydrogen The paper again emphasizes hydrogen studies that can be useful in determining corrosion in boilers. As a result, even in 1990, hydrogen analysis was being presented as the primary way of determining boiler corrosion. However, as earlier stated, hydrogen evolution as a means of measuring corrosion suffers for the complications earlier cited, that is hydrogen atom reactions with iron oxides, hydrogen gas diffusion into metal substrates and problems of determining the source of the hydrogen measured as being from corrosion breakdown of chemical additives, or similar sources as mentioned above or as mentioned in the articles cited above.

Each of the articles cited above are incorporated herein by reference.

In attempting to determine what other techniques might be available to measure iron in boiler waters, the following articles and/or patents have come to our attention:
1. An article in Analytical Chemistry, Volume 34 (3) March 1962, pages 348-352, which article teaches the use of 4,7-diphenyl-1,10-phenanthroline for the purpose of determining ferrous ion in the presence of ferric ion. This phenanthroline reagent apparently specifically forms complexes with ferrous ion which complexes have a different absorption spectra than the complexes formed by ferric ion. Although iron corrosion is mentioned in general, there is no mention of boiler waters in this reference.
2. In Analyst, August, 1975 (Volume 100), pages 549-554, Fadrus, et al, teaches a method for determining ferrous ion in water in the presence of ferric ion again using 1,10-phenanthroline. Fadrus teaches that ferric ion complexation does provide interferences and he masks this interference with what he terms "complexones". His recommended complexone is nitrilotriacetic acid. Again. Fadrus, et al, are concerned primarily with the analytical chemistry of ferrous and ferric phenanthroline complexes and does not use or teach the use of this chemistry in boiler waters.
3. In US Patent 3,836,331, Stookey teaches the use of 3-(2-pyridyl)-5,6-bis(4-phenylsulfonic acid)-1,2,4-triazine (alternatively : 3-(2-pyridyl)-5,6-diphenyl-1,2,4-triazine-p,p'-disulfonic acid, usually monohydrate) (hereinafter "FZ") and certain salts thereof, particularly the sodium salts, as useful reagents for spectrophotometric determinations of iron in water and in other solutions. Stookey, in column 1, does speak of the necessity to analyse for iron content in boiler waters because the iron oxide content of the water is an index of the rate of corrosion taking place in the boiler. He then however teaches that conventional methods for analysis of iron normally involve collecting iron compounds, solubilizing these compounds by adding for example hydrochloric acid and allowing sufficient time for dissolution, and then adding a reducing agent such as hydroxylamine or hydroxylamine hydrochloride to reduce the iron to the ferrous form. He then specifies the use of FZ as a specific complexing agent for ferrous ion. This FZ reagent acts as a complexing agent for ferrous ion, ferric ion, cobalt salts, copper salts, and the like. His remaining teachings emphasize the use of FZ as a ferrous ion chromogen. His teachings include procedures for monitoring total iron in the standard procedures he outlines. These procedures involve collecting samples, dissolving samples, reducing samples specifically by the addition of reducing agents such as hydroxyl ammonium chloride, adding reagents suitably buffered, and measuring iron content by colorimetric means.
4. US Patent 3,770,735, Stookey, again describes the FZ compounds but primarily emphasizes the synthesis of these compounds. No mention of actual use in boiler waters is made except by hypothesis that boiler water iron content may be an example where such an analysis might be necessary.
   However, Stookey speaks of the iron oxide content of the water as an index to the rate of corrosion taking place in the boiler and this iron oxide content can include both forms of ferrous and ferric ion and can include as indicated in some of the earlier references cited, quite complex admixtures of various types of iron oxides, such that the measurement of iron oxides is not a measure of corrosion occuring in real time in boilers.
5. In Talanta, Volume 31, No. 10A, pages 844-845, 1984, Li Shi-Yu et al, described the use of ion exchange colorimetry to determine microamounts of iron in water with FZ reagent. The solution studied came from drinking water supplies.
6. In Analytical Chemistry, Volume 42(7) June 1970, the advantages of using the FZ chemical reagent in combination with a digestion procedure for the determination of total iron in various waters is taught. The waters studied were well waters obtained in Ames, Iowa. No mention of boilers is made.
7. Finally, an article appearing in Analytical Chemistry, Volume 48 (8) July 1976, pages 1197-1201, provides for characterization and application of the FZ iron reagent as a ferrous ion indicator. In this paper a number of references are given to the use of the reagent and the combination of the reagent in a number of automated analyzers and in a test kit for determination of ferrous ion, primarily in blood serum. In a list of references cited by the author, Gibbs, the use of the FZ reagent in potable water, sea water, plant nutrients solutions, plant materials, high purity reagent chemicals, bathocuproine sulfonate analysis, as well as in the use to determine cobalt, ruthenium, osmium and other metals are listed. These various analytical uses take advantage of the fact that the ferrous ion complex with FZ is colored and the ferric ion-FZ complex is not colored. Using this characteristic, the FZ reagent has also been used to indirectly detect the presence of ascorbic acid in fruit juices, in blood serum and urine, to detect the presence of sulfur dioxide in various liquid samples, and after absorption from various gases, and various enzymatic activities in the NADH/NAD redox systems. No use in boilers is summarized therein.

It is an object of our invention to provide for a method of determining the presence of actual corrosion of boiler system ferrometals in contact with boiler waters.

It is another object of the invention to determine actual corrosion rates of boiler system ferrometals e.g. carbon steels, stainless steels, iron, or other iron alloys, in contact with boiler waters containing boiler water treatment chemicals such chemicals including, but not necessarily limited to, oxygen scavengers such as bisulfite, hydrazine, carbohydrazide, erythorbic salts, and the like; and such other chemical treatments such as hardness transporting chemicals including EDTA, HEDTA, NTA, and certain polymeric complexes, as well as other boiler water treatment chemicals such as the precipitating treatments for hardness, that is phosphates, phosphonates, and the like.

We aim to determine actual corrosion rates in various boiler system units containing ferrometals in contact with boiler waters, either containing boiler water treatment chemicals or not containing boiler water treatment chemicals (such as in the condensate systems) by obtaining a known aliquot volume of a boiler water sample in a manner which protects this sample from oxygen or any oxidizing species, then adding this aliquot sample to a solution of FZ reagent to form a soluble complex with the FZ reagent, i.e. 3-(2-pyridyl)-5,6-diphenyl-1,2,4-triazine-p,p'-disulfonic acid, or its salts. After formation of the ferrous ion/FZ complex, this complex is relatively stable to oxidation, however, the presence of the complex should he measured spectrophotometrically by measuring, within a reasonable time period of from about 1 minute to about 48 hours, the absorbance of a known standard containing this complex and comparing that with a concentration of an unknown sample having known aliquot relationships at a wave length of about 560 ± 30 nanometers.

It is an object of this invention to then calculate the amount of ferrous ion present in the boiler waters by making such a comparison of absorbance of known samples and to continuously measure this concentration by repeating the sampling steps, the formation of the complex steps, the spectrophotometrically measuring steps, and the calculating steps in a manner to provide for the measurement of actual corrosion rates of boiler system ferrometals in contact with boiler waters.

Our invention is a method of determining actual corrosion rates of boiler system ferrometals in contact with boiler waters containing or not containing boiler water treatment chemicals a preferred embodiment of which comprises:
Step 1. Obtaining a known volume of a boiler water sample from a boiler system unit in a manner which protects said sample from oxygen, or an oxidizing chemical:
Step 2. Then adding said known volume of said sample to a known volume of an acetate buffered standardized solution of 3-(2-pyridyl)-5,6-diphenyl-1,2,4-triazine-p,p'-disulfonic acid monohydrate, or salts thereof,and mixing to obtain a solution of a water soluble ferrous ion/FZ complex: and then
Step 3. Placing this solution of soluble ferrous ion/FZ complex into a photometric cell of prescribed length and spectrophotometrically measuring the absorbance of said ferrous ion/FZ complex at a wave length of about 560 ± 30 nanometers; and then
Step 4. Calculating the amount of ferrous ion present in said solution of ferrous ion/FZ complex by comparing the absorbance obtained in Step 3 with the absorbance measured by generating a standard ferrous ion/FZ absorbance curve which curve is previously generated using known concentrations of ferrous ion from 1 to about 1000 parts per billion; and then
Step 5. Repeating steps 1-4, as necessary to determine actual corrosion rates over a known time period.

The FZ complexing agent has the following formula:

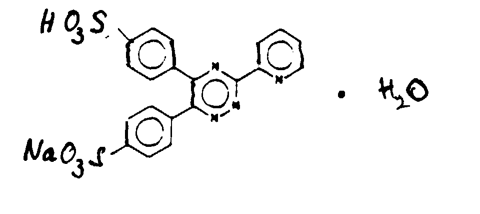

This complexing reagent is primarily used as the monosodium salt but may also be made up in other salt forms. The complex is primarily present as a monosodium salt having one water of hydration related thereto and can be commercially purchased from the Hach Company. The complexing agent should be used at a known pH of from about 2 to about 6, preferably from between about 3 and about 5 and most preferably at a pH of approximately 3.6 ± 0.2. The standardized solution of the FZ reagent primarily is a solution of this above FZ complexing agent that preferably contains an acetate buffer such that the pH ranges between 3.6 ± 0.2, although other buffering systems besides the acetate buffering system may be used

As stated above, it is critical to obtain and protect the boiler water sample from oxygen or any oxidizing media. This is because the initial reactions involving corrosion of ferrometals in contact with boiler waters creates ferrous ions. Subsequent reactions of ferrous ion with oxygen or other chemicals provide for quite complicated forms of other iron salts some of which or all of which are insoluble in water and either form as scales or as precipitates in the boiler water. Therefore the initial samples taken from each boiler system unit must be taken carefully in a manner to protect the sample once taken from the system from oxygen or from oxidizing species.

The known volume, or aliquot, sample is immediately added to a known volume of the FZ reagent solution, buffered as above. Preferably an acetate buffer solution as described above is used. Although an acetate buffer is used primarily in our technique, any other buffer in the range between about 2.0 and 6.0 would be sufficient. Buffering ingredients which might form precipitates with boiler chemicals or contaminants should be avoided Again, it is preferable that during the collection of the initial boiler water sample, protected from oxygen or oxidizing chemicals as above described, with the FZ reagents, which reagents should also preferably be oxygen free, the complex solutions formed be protected from oxygen, although these complexes are considerably more stable against oxygenation than are the original ferrous containing solutions. It is advisable to maintain some protection against exposure to air or oxygen during the entire process of our invention

The reaction between ferrous ions in the boiler water samples as taken and described above and the FZ reagent solutions as described above are nearly instantaneous. However, it is advisable to provide some mixing and some reaction time to assure oneself that a homogeneous solution of the water soluble ferrous ion/FZ complex has been obtained prior to measurement of its concentration spectrophotometrically.

The spectrophotometric measurement is fairly straightforward and can use any equipment available which has the capability of presenting a light source having a wave length of about 560 ± 30 nanometers through a controlled length cell path into which the ferrous ion/FZ complex solution has been added, and which equipment includes means for measuring light absorbance, light transmission and the like, of this wave length, through the spectrophotometric cell containing the ferrous ion/FZ complex solution.

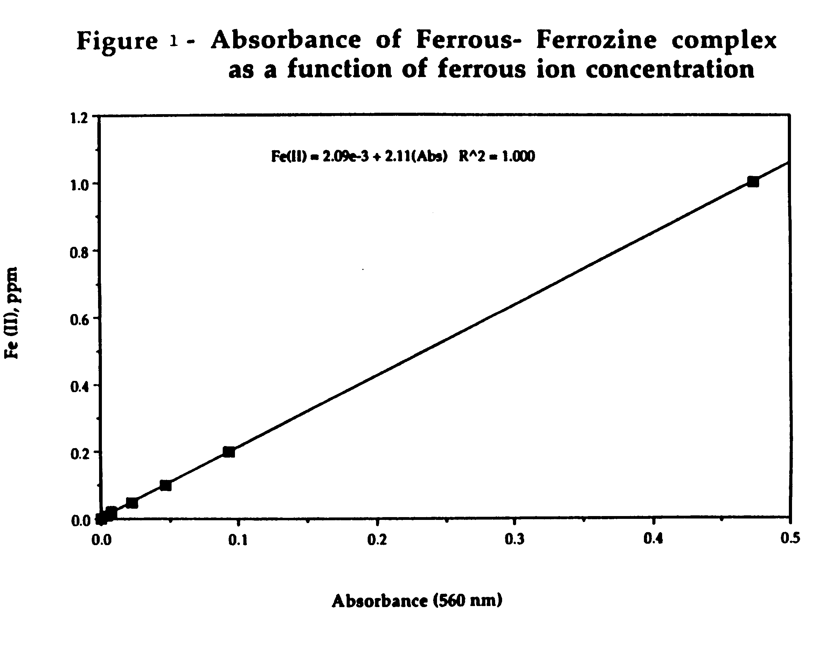

After measuring absorbance of the ferrous ions/FZ complex at the appropriate wave length taught above, it is easy to calculate the amount of ferrous ion present in the boiler water sample by comparing this absorbance with a standard absorbance obtained by a standard absorbance/concentration curve obtained by measuring different concentrations of ferrous ion solutions and the Fe(II)/FZ complex Such a standard curve is presented below as Figure 1 and describes the absorbance at 560 nanometers of ferrous ion FZ complex as measured according to the techniques of this invention.

To measure the corrosion rates of boiler system ferrometals in contact with boiler waters, particularly those boiler waters containing treatment chemicals, it is advisable that the FZ complex be present in the standardized solution to which the boiler water sample is added in sufficient concentration to overwhelm any additional complexes or reactions that might be occuring or which might have already occurred between ferrous ion as generated by the corrosion reactions, and whatever treatment chemicals are present. For example, it is known that ethylene diamine tetraacetic acid, ie. EDTA, a known boiler water treatment chemical can form complexes with ferrous ion. It has been shown that these complexes can interfere with the reaction with the FZ reagent However, by increasing the concentration of the FZ reagent to a sufficiently high concentration, this interference is overwhelmed and the equilibrium forming the ferrous ion EDTA complex is no longer controlling. Since boiler waters may be treated with EDTA, and such other complexing reagents as nitrilotriacetic acid, for the purpose of controlling hardness formation and hardness scales, it is unlikely that EDTA or NTA or like complexing agents are going to be present in the boiler waters sampled in concentrations much above from about 0.5 to about 50 parts per million, even though those boiler waters are being treated specifically by formulations containing these strong complexing agents. As a result, it has been found that the reagent containing the FZ complexing agents should provide for essentially quantitative formation of ferrous ion FZ complexes in the presence of concentrations of the FZ complexing agent ranging from approximately 0.005 to about 0.20 molar concentration of FZ complexing agent in the acetate buffer solution. A preferred concentration of the FZ complexing agent in this buffered solution, in the absence of EDTA/NTA, or like chemical complexing agents, is at about 0.025 molar, although higher concentrations are permitted. Higher concentrations of FZ reagent is needed in the presence of EDTA, for example, see Table II.

The acid buffer, as earlier stated, should control the pH in a range from between about 2 to about 6, preferably from between about 3 to about 5, and most preferably at a pH of between about 3.6 ± 0.2.

The boiler system can contain a number of different boiler system units, each unit subject to corrosion according to the reactions cited in the references incorporated herein by reference and presented above. The primary reaction for corrosion is the reaction of iron metal with water to form ferrous ion, and a similar reaction of the protons in water to form hydrogen gas, the reactions occurring at the anodic and/or cathodic sites respectively. If samples are taken of the boiler waters immediately within the boiler water system being monitored, and the samples are protected from oxygen or oxidizing species, and the samples are added immediately to the FZ reagent solutions as described above, the complex formed is sufficiently stable to permit its spectrophotometric measurement. Therefore the direct measurement of instantaneous corrosion occurring with the boiler system or the boiler system unit being monitored is now available, with no drawbacks as in hydrogen measurement.

These boiler system units can include but are not necessarily limited to the inlet and/or outlet of an economizer, the inlet and/or outlet of the pre-heaters, the inlet and/or outlet of each steam condensers, the actual feed water to the boiler, the actual blow down from the boiler, the inlet and/or outlet samples obtained from any storage vessel or any holding tank or any aqueous condensate receiver, any steam receiver which is undergoing condensation to provide a liquid sample therein, any heat exchanger, any working unit being driven by the steam generation such as a turbine, a piston and the like. Also, samples from the water wall header and outlet of the deaerator, inlet and outlet of the water treatment system providing for low hardness water, such treatment facilities including ion exchange and the like may be monitored for corrosion. Also, water samples in any line, any pump, any vessel, and the like, which devices are contained in a boiler system may be so monitored.

Although total iron analysis has been run and hydrogen analysis has been run in an attempt to detect direct corrosion, these procedures have fallen by the wayside either because of complications in measuring corrosion rates in the field or because of the unsureness that the corrosion rates that were measured depended in fact upon the chemicals species being measured. By direct measurement of ferrous ion in boiler water samples which samples are being collected directly from boiler water units within an operating boiler, this unsureness is eliminated.

To provide for better understanding of our invention, the following examples are given.

### EXAMPLES

To better exemplify our invention the following tests are presented.

Reagents were made as follows:
A. Into a one liter volumetric flask, 72.12 of sodium acetate, as the trihydrates, and 274.29 milliliters of glacial acetic acid were added with sufficient distilled water to bring to volume. The resulting solution is buffered at a pH of 3.6 ± 0.2.
B. 50 milliliters of the above buffer solution was added to a 100 milliliter volumetric flask to which was added 1.28 of FerroZine®¹ reagent. FerroZine® is an analytical quantitative reagent obtained from the Hach Chemical Company, Ames, Iowa. The FerroZine® reagent is the monosodium salt, as a monohydrate, of 3-(2-pyridyl)-5,6-bid(4-phenysulfonic)-1,2,4-triazine. The flask is then brought to volume with the buffer solution. This FZ/reagent solution contains 0.025 molar concentration of the FZ complexing agent and is buffered at a pH of 3.6 ± 0.2.
C. A 100 part per million standard solution of ferric ion was prepared by adding 0.860 grams of ferric ammonium sulfate, 12 hydrates, to a small beaker to which was also added 4 milliliters of analytically pure nitric acid and approximately 50 milliliters of distilled water. The solution was heated to a gentle boil until all of the solids had dissolved The solution was cooled and another 6 milliliters of ultrapure nitric acid was added. The contents of the beaker were analytically transferred to a one liter volumetric flask and made up to volume with distilled water. The solution so prepared is 100 parts per million in ferric ion concentration.
D. A standard solution containing 100 parts per million ferrous ion was made up by adding 0.701 grams of ferrous ammonium sulfate, 6 hydrates, to a 1 liter volumetric flask and adding sufficient distilled water to bring to volume. This resulting solution contains 100 ppm ferrous ion concentration and is protected from oxygen or oxidizing chemicals and used as soon as it is prepared.

FerroZine® is a registered trademark of Hach Chemical Company.

Using the above solutions, a standard absorbance - concentration curve was generated for the determination of ferrous ion in solution. A series of 50 milliliter volumetric test tubes had 1 milliliter of the FZ reagent added thereto. A blank solution was prepared by diluting with distilled water to the 50 milliliter mark ad various standard concentrations of ferrous ion were made by adding different concentrations of the ferrous ion standard solution to these test tubes and bringing to volume with the standard FZ reagent solution Color development of the ferrous ion/FZ complex occurred over a period of from about 1-10 minutes and then the absorbance of each of these solutions contained in a photometric cell of known rate were measured at a wavelength of 560 nanometers. The standard absorbance curve is presented in Figure 1. This standard absorbance curve is essentially unchanged, even in the presence of ferric ion, since the ferric ion - FZ complex does not absorb light of this general 560 ± 30 nanometer wavelength. (See the results in Table I.)

Samples of boiler waters were obtained from various operating boiler system units by taking the water sample in a manner that guaranteed the sample was protected from exposure to oxygen or oxidizing systems. Samples were taken in such a manner so as to provide a known volume of the boiler water samples and the sample was added immediately to the standard solution of FZ reagent as prepared above. This was also done in a manner to protect both the FZ solution and the boiler samples from exposure to air, any oxygen sources, or any oxidizing chemicals.

For each of these samples, the color development is completed within one minute, or earlier, at temperatures ranging from about 60 to about 80°F. The complex is stable to air oxidation for at least 48 hours. In spite of the oxygen stability for the ferrous/FZ complex, it is suggested that all measurements and sampling be made in an oxygen protected or free environment. It is important that the sample to be analysed from the boiler system unit be collected in a testing apparatus such that the sample is mixed with a solution of the FZ reagent, both sample, and preferably the reagent also, being protected from oxygen exposure, air exposure and the like.

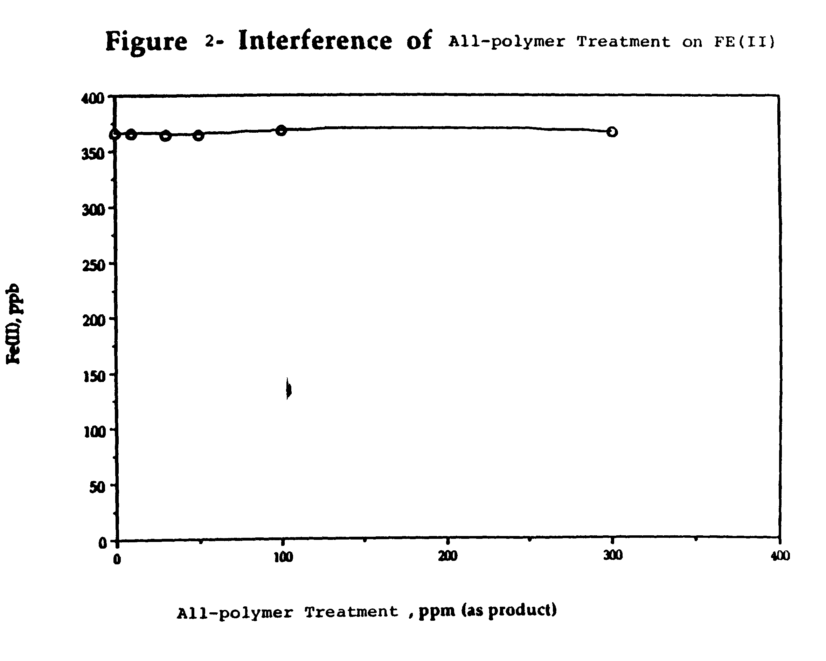

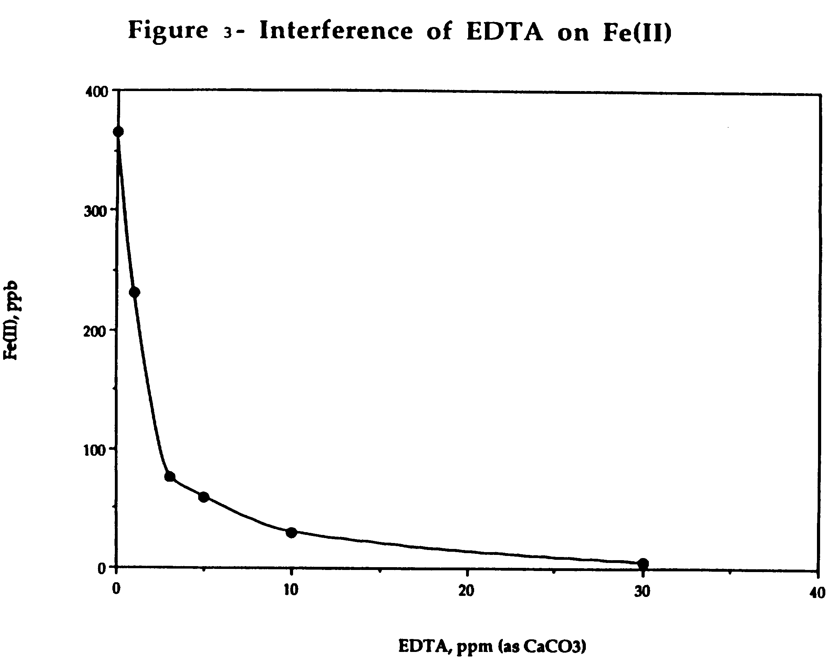

Prior to presenting the results of actual boiler unit tests, it was determined that ferrous ion in solution may be affected by the presence of certain internal boiler water chemical treatments. Towards this end, various water samples containing calculated amounts equaling 370 parts per billion ferrous ions, were spiked with these boiler water chemicals prior to admixture with the FZ reagent solution. A polymeric transport chemical provided by Nalco Chemical Company was spiked at a concentration up to 300 ppm based on total product. Also, a solution of ethylene diamine tetraacetic acid, as partial sodium salt, were also spiked into the solution containing ferrous ions. Figure 2 and Figure 3 provide the results of these tests.

In the presence of the polymeric transport chemical obtained from Nalco Chemical Company, and sold under the trade name "Transport Plus"®², no negative effect was obtained for the determination of ferrous ion in solution. However, in the case of ethylenediaminetetraacetic acid, even at a concentration equal only to 1 ppm EDTA, calculated as calcium carbonate, ferrous ion determinations were drastically affected. At an EDTA dosage of 30 ppm, as calcium carbonate, ferrous ion concentration detected by FZ reagent was below the detection limit.
²Transport Plus® is a registered trademark of Nalco Chemical Company.

This interference can be eliminated by increasing the concentration of the FZ reagent as is shown in Table II, so that when the concentration of the FZ reagent is high enough compensation for the interference cause by the ethylenediaminetetraacetic acid chelant is achieved.

Therefore, a key to monitoring for the presence of corrosion products, as measured by the presence of ferrous ion in boiler waters, particularly when those boiler waters are treated with certain boiler water treatment chemicals, is to have sufficient and effective concentrations of the FZ reagent present to overwhelm and compensate for any interferences caused by the boiler chemical treatment chemicals.

The required concentration of FZ reagent to compensate for the presence of ethylenediaminetetraacetic acid is presented in Table II.

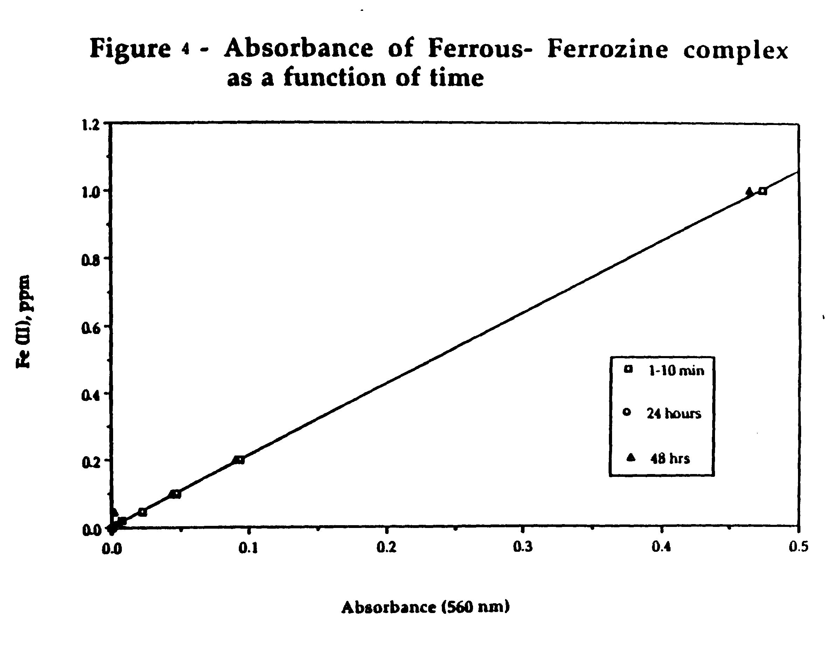

The time for development of the ferrous ion FZ complex is given in Figure 4. As can be seen from this Figure, the color development is complete within 1-10 minutes and is remarkably stable for a period of at least 48 hours.

Using the above tests, and sufficient FZ reagent to overwhelm any complexation with boiler treatment chemicals such as EDTA, boiler feed water ferrous ion pick up studies

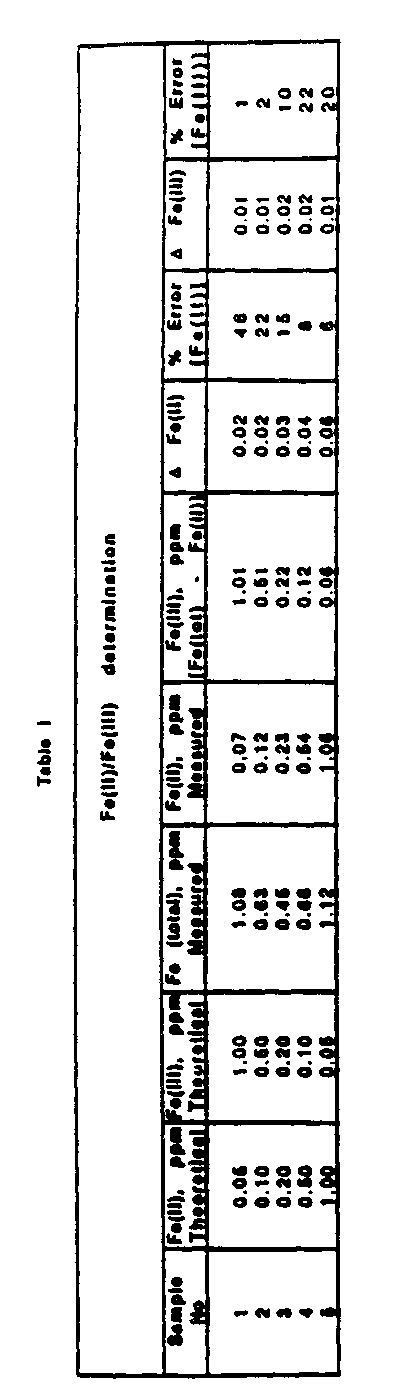

**Table II**

| Required concentration of ferrozine reagent to compensate for EDTA interference (Fe(II) = 300 ppb) | |
|---|---|
| EDTA Concentration (as CaCO₃) | Ferrozine [M] |
| 0 | 0.025 |
| 2 | 0.071 |
| 20 | 0.141 |

were made across various boiler system units operating in a steel manufacturing plant. Samples across an economizer operating within this boiler were analyzed using the proprietary ferrous iron FZ sampling and analysis procedures above. Chemical treatment programs containing ethylenediaminetetraacetic acid as well as chemical treatment programs containing nitrilotriacetic acid and another chemical treatment program containing an all polymer transport treatment chemical were evaluated

Tests confirmed that corrosion across the economizer boiler unit was a direct function of the dosage of strong chelant chemical treating agents.

With no feed of boiler treatment chemical containing EDTA, or NTA, but with boiler treatment chemicals containing oxygen scavengers such as carbohydrazide and erythorbic acid salts, the ferrous ion pickup levels were 0 parts per billion However, when operating with boiler treatment chemicals used primarily for the purpose of transporting and controlling hardness scales and hardness scale creating chemicals such as magnesium, calcium, silicates, and the like, ferrous ion determinations were directly increased as the dosage of the chemical chelant was increased. Ferrous ion levels were reported as high as 162 parts per billion when an EDTA containing formulation was added so as to retain 0.2 ppm residual of the EDTA containing formulation. Ferrous ion levels were detected (with treatment at a 0.1 ppm EDTA residual dosage) at concentrations ranging from 78-85 parts per billion with or without the presence of the above mentioned oxygen scavengers. Iron pickup levels using a program of boiler treatment chemicals presented as the Betz Company's B1986 program, which program led to dosages of a 0.1 ppm formula weight (B1986) residual, equalled 101 parts per billion ferrous ion, in the absence of either carbohydrazide or erythorbate. In the presence of a chemical treatment program in which the hardness complexing agent was nitrilotriacetic acid (NTA), ferrous ion was detected at 42 parts per billion when the NTA was added to the boiler system at a residual of 0.1 ppm.

During the above tests, dissolved oxygen levels in the feed water were consistently controlled at 0 parts per billion.

Since corrosion is a direct function and is demonstrated directly by the presence of freshly generated ferrous ion, it is believed that this technique of monitoring boiler waters, particularly in the presence of boiler water treatment chemicals, using the techniques which provide for adequate concentrations of FZ reagent so as to compensate and overwhelm any complexes formed by chelating agents present in the treating chemicals, such chelating agents demonstrated to include EDTA and NTA. At least under the conditions of these tests, polymeric transport agents such as the Transport Plus® chemical supplied by Nalco Chemical Company do not cause corrosion.

Also, based on the results recited above, the formulations containing nitrilotriacetic acid were not nearly as aggressive in terms of creating corrosion of iron across this economizer as were the formulations containing ethylenediaminetetracedic acid.

In another series of tests, chemical treatment programs used typically to treat boiler waters, which programs contained ethylenediaminetetraacetic acid and various polymers used as dispersants also were demonstrated to show iron pickup in these boiler samples in the form of ferrous ion which could he monitored using the ferrous ion/FZ proportional monitoring techniques of this invention Again, corrosion potential, as measured by the concentrations of ferrous ion determined in boiler waters being treated with these chemical treatments, appeared to increase directly as the concentration of the ethylenediaminetetraacetic acid increased Earlier tests using polymers indicated that the polymers had no essential effect under these test conditions.

Table III presents the results obtained when this operating economizer was monitored using the techniques of this invention for iron pickup.

As can be observed, the data in Table III also presents the total iron found in the economizer boiler samples. This total iron was remarkably close to the ferrous ion in

**Table III**

| Economizer Iron Pickup Study | | | | |
|---|---|---|---|---|
| Chelant | FW Chelant Dosage (ppm) as CaCO3 | FW Chelant Residual (ppm) as CaCO3 | Economizer ΔFe(II) ppb | Economizer ΔFe(Total) ppb |
| EDTA | 0.35 | 0.30 | 152 | 159 |
| EDTA | 0.14 | 0.09 | 85 | 85 |
| EDTA | 0.14 | 0.09 | 78 | 84 |
| EDTA | 0.35 | 0.30 | 120 | 136 |
| EDTA | 0.30 | 0.25 | 151 | 138 |
| none | 0.00 | 0.00 | 0 | 4 |
| NTA | 0.30 | 0.25 | 96 | 93 |
| NTA | 0.15 | 0.10 | 42 | 43 |
| EDTA | 0.25 | 0.20 | 128 | 125 |
| none | 0.00 | 0.00 | 1 | - |
| EDTA | 0.24 | 0.00 | 85 | - |
| EDTA | 0.24 | 0.16 | 133 | |
| EDTA | 0.24 | 0.19 | 156 | 163 |
| EDTA | 0.24 | 0.19 | 162 | - |
| EDTA | 0.24 | 0.19 | 149 | - |
| EDTA | 0.07 | 0.02 | 48 | 48 |
| EDTA | 0.15 | 0.10 | 101 | 102 |
| 1. ΔFe(II) or ΔFe(Total) = Fe(II) or (Total) at the economizer outlet - (minus Fe(II) or (Total) at the economizer inlet 2. Fe_{(Total)} = Fe_{II} + Fe_{(III)} | | | | |

practically all cases and demonstrates that the iron presence in the economizer boiler waters was not present because of contamination scaling, or residual iron but in fact was present almost exclusively from the formation of ferrous ion in a corrosive mechanism.

Further tests were completed on line at various treatment levels for each of the EDTA containing NTA containing or all-polymer treatment programs normally found in the industry. Table IV presents the data obtained when this boiler system was operated with the listed percentages of recommended dosages for various formulations which contained, as the primary hardness treatment reagents, EDTA, NTA, or all-polymer treatment formulations. The data for EDTA, #1, was obtained from a treatment program suggested by one water treatment chemical company, EDTA #2, was from a treatment program suggested by that same company but having a slightly different formulation, EDTA, #3 was obtained from a second water treatment chemical company and the NTA and all polymer programs were also commercially available products.

It is to be noted that the dosages recommended by each of these companies, when used at less than 100 percent of the recommended level, can result in boiler scale problems derived from hardness scales. Therefore, it is clear that one would want to eliminate not only the scale problems but also to provide an anti-scaling program which would not be corrosive to a boiler ferrometals.

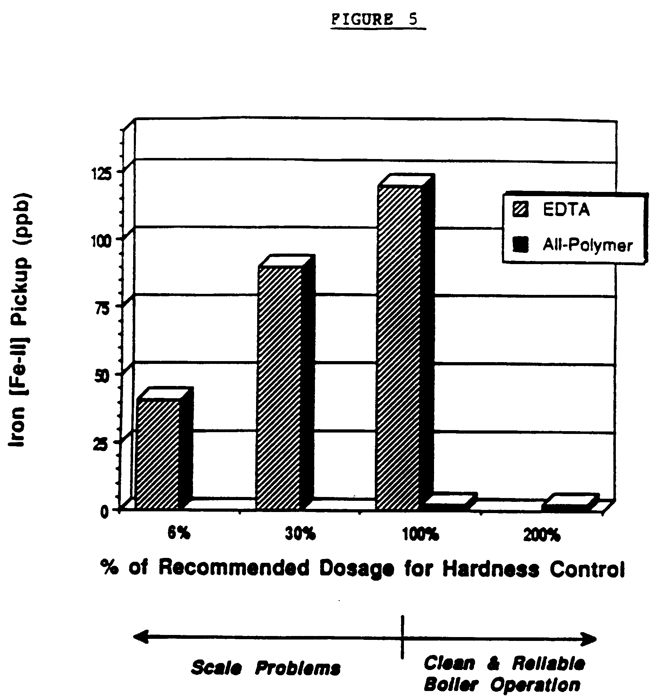

Figure 5 presents the iron pickup, as measured by ferrous ion/FZ complexation, when an EDTA based treatment program is compared to an all polymer treatment program for scale control. The recommended dosages for hardness control using EDTA are averages of treatment systems recommended by two separate boiler treatment chemical companies. By measuring ferrous ion according to the techniques described and claimed herein, it is clear that the all polymer program can provide both corrosion free environments and hardness scale transport and hardness scale minimization.

**Table IV**

| Economizer Iron Pickup Study | |
|---|---|
| % Recommended Dosage for Hardness Control* | Fe-II Economizer Pickup |
| EDTA #1 | |
| 100% | 152 |
| 30 | 85 |
| 30 | 78 |
| 100 | 120 |
| 83 | 151 |
| 67 | 128 |

| EDTA #2 | |
|---|---|
| 53 | 133 |
| 63 | 156 |
| 63 | 162 |
| 63 | 149 |

| EDTA #1 | |
|---|---|
| | 30 |
| 8 | 45 |

| BASELINE (with Erythorbate Oxygen Scavenger) | |
|---|---|
| 0 | 0 |
| 0 | 1 |

| EDTA #3 | |
|---|---|
| 7 | 48 |
| 33 | 101 |

| NTA | |
|---|---|
| 83 | 96 |
| 33 | 42 |

| ALL-POLYMER | |
|---|---|
| 100 | 2 |
| 200 | 2 |

### AUTOMATED ANALYSIS, USING FZ-FERROUS COMPLEX

An automatic system has been constructed that provides samples from any sampling point within a boiler operating unit or within any boiler system operating unit. These boiler operating units can include those listed previously, including the boiler itself, the down comer tubes, the overhead condensation system, or any condenser therein, the economizers, the pre-heaters, the ion exchange water preparation units or any line, water or steam vessels, tanks, pumps, or the like. This automatic monitoring system includes mechanical and electronic devices which will allow the operation, automatically under the control of a microprocessor, of all functions required to perform our tests. The functions provided by this device and controlled by this device include sample manipulation, such manipulation including the taking and transporting of samples in an oxygen free environment to a reaction chamber where the samples are reacted with the FZ reagent, appropriate time permitted for formation of the ferrous ion/FZ complex, then transport of this solution containing that ferrous ion/FZ complex into a photometric cell. The automatic device also provides for the generation of a light source wavelength of approximately 560 nanometers, the measurement of absorption of this light when passed through the optical cell containing the ferrous ion/FZ complex, the automated reading of this absorbance signal, and the data processing necessary to provide for read outs which are measurable and proportional to the concentration of ferrous ion in the sample taken This microprocessor also would control signal generation, data processing, and would control component failure detection.

This system is capable of being programmed, calibrated, and data acquisition can be implemented easily using this system both in the laboratory and in field installed devices. Data generated can be down loaded onto any computer having appropriate interface with the microprocessor, which is part of the analyzer. This analyzer is subject to another patent application which is copending with this application entitled "On-Line Analyzer for Ferrous Ion" by Banks, et al, which application is incorporated herein by reference. This proposed analyzer provides for controlled volume samples being withdrawn from the sample source line without air contact, transport of samples into the instrument manifold via a miniature positive displacement pump. (However, any means of transporting a boiler water sample without air contact into an instrument manifold may be used). A sample blank reading is taken and buffered FZ reagent is injected at a volume ratio of from about 100:1 to about 1:100, depending on concentrations of FZ reagent and iron, preferably from 50:1 to about 1:50, into the sample stream and allowed to react. Another reading of the photometric transmission and/or absorbance of the reacted sample is taken and, in combination with the blank value, is converted into parts per billion ferrous ion via software calculations based on standardized absorbance concentration determination made earlier, then displayed either on a terminal or stored in memory for later downloading. Calibration of the instrument can be done manually at any time by employing known ferrous ion standards and programming proper factors.

The proposed analyzer is designed to determine the concentration of ferrous ion in boiler water samples obtained from any boiler water unit or boiler system unit within the range of 0.4 to 1000 parts per billion ferrous ion in the boiler water sample.

## Claims

1. A method of determining actual corrosion rates of boiler system ferrometals in contact with boiler waters containing boiler water treatment chemicals, which method comprises:
(1) Obtaining a known volume of a boiler water sample in a manner which protects said sample from oxygen; and
(2) Adding said known volume of said sample to a known volume of an acetate buffered, at pH from 2.0 to 6.0, solution containing an effective concentration of 3-(2-pyridyl)-5,6-diphenyl-1 2,4-triazine-p,p'-disulfonic acid monohydrate reagent or salt thereof ("FZ") and mixing; thereby forming a soluble ferrous ion/FZ complex solution; and then
(3) Measuring spectrophotometrically the absorbance of said ferrous ion/FZ complex at a wavelength of about 560 ± 30 nanometers; and then
(4) Calculating the amount of ferrous ion present by comparing absorbance obtained in step (3) with a standard ferrous ion/FZ absorbance curve previously generated; and then
(5) Repeating steps 1 to 4, as necessary, to determine actual corrosion rates over a known time period.

2. A method according to claim 1 wherein the standardized solution of 3-(2-pyridyl)-5,6-diphenyl-1,2,4-triazine-p,p'-disulfonic acid reagent contains an effective amount of said reagent to form said ferrous ion/FZ complex in essentially quantitative amounts, even in the presence of said boiler-treatment chemicals.

3. A method according to claim 1 or claim 2 whereby the boiler water sample is taken from a boiler system unit which is an inlet and outlet of steam condensers, feedwater to the boiler, blowdown from the boiler, or inlet and outlet from any storage vessel, holding tank, receiver, heat exchanger, turbine, piston, water wall header, outlet of the deaerator, and any combination thereof; and the results of ferrous ion determination at each such inlet and outlet are compared, thereby obtaining a determination of corrosion rates in the given such boiler system unit.

4. A method according to any one of claims 1, 2 or 3 wherein the methods of obtaining said boiler water sample, of adding same to the FZ reagent, of measuring absorbance of ferrous ion/FZ complex and comparing with a standard curve to determine and calculate amount of ferrous ion present in said sample are performed automatically and continuously.

5. A method according to any one of the preceding claims wherein the standardized FZ reagent has a pH of from 3.4 to about 3.8 and contains at least 0.005 moles per liter of FZ reagent.

6. A method according to any one of the preceding claims wherein the volume ratio of boiler water sample to standardized FZ reagent is between 100:1 to 1:100.

7. A method according to any one of the preceding claims wherein the ferrous ion concentration measured ranges from between 0.5 to 1000 parts per billion, based on total boiler water sample volume.

8. A method according to any one of the preceding claims wherein after the measurement of absorbance of ferrous ion/FZ complex in the boiler water sample is completed, a known amount of a strong reducing agent which is hydroxylamine hydrochloride, thioglycolic acid or its salts, erythorbic acid or its salts, hydrazine, carbohydrazide, sodium bisulfite or mixtures thereof is added to the ferrous ion/FZ complex solution, mixed and reacted for a sufficient time to reduce any ferric ion species present to ferrous ion, which ferrous ion reacts with residual FZ reagent forming additional ferrous ion/FZ complex solution, then remeasuring the absorbance of this additional Fe(II)-FZ solution, thereby measuring total iron content, and then obtaining ferric ion content by subtracting the original ferrous ion content from total iron content.

9. A method according to claim 8, wherein the strong reducing agent is thioglycolic acid.

## Patentansprüche

1. Verfahren zum Ermitteln der tatsächlichen Korrosionsraten von Kesselsystem-Ferrometallen, die mit Kesselwasserbehandlungschemikalien enthaltenden Kesselwässern in Kontakt stehen, welches Verfahren umfaßt:
(1) das Ziehen einer Kesselwasserprobe bekannten Volumens auf eine Weise, bei der die Probe vor Sauerstoff geschützt ist; sowie
(2) das Zugeben dieser Probe bekannten Volumens zu einem bekannten Volumen einer auf einen pH von 2,0 bis 6,0 acetat-gepufferten Lösung, die eine wirksame Konzentration eines 3-(2-Pyridyl)-5,6-diphenyl-1,2,4-triazin-p,p'-disulfonsäuremonohydrat-Reagens oder eines Salzes davon ("FZ") enthält, und das Vermischen; wodurch eine Lösung eines löslichen Eisen(ll)ion/FZ-Komplexes gebildet wird; und dann
(3) das spektralphotometrische Messen der Absorption des Eisen(ll)ion/FZ-Komplexes bei einer Wellenlänge von etwa 560 ± 30 nm; und dann
(4) das Berechnen der vorhandenen Eisen(II)ionen-Menge durch das Vergleichen der in Schritt (3) erhaltenen Absorption mit einer zuvor hergestellten Eisen(II)ion/FZ-Absorptionsstandardkurve, und dann
(5) das Wiederholen der Schritte 1 bis 4, wie erforderlich, um die tatsächlichen Korrosionsraten über eine bekannte Zeitspanne zu ermitteln.

2. Verfahren nach Anspruch 1, worin die standardisierte 3-(2-Pyridyl)-5,6-diphenyl-1,2,4-triazin-p,p'-disulionsäurereagenslösung eine wirksame Menge dieses Reagens enthält, um den Eisen(ll)ion/FZ-Komplex auch in Gegenwart der Kesselbehandlungschemikalien in im wesentlichen quantitativen Mengen zu bilden.

3. Verfahren nach Anspruch 1 oder 2, bei dem die Kesselwasserprobe aus einer Kesselsystemeinheit gezogen wird, die ein Ein- und Auslaß von Dampfkondensatoren, Kesselspeisewasser, Blowdown vom Kessel, oder Ein- und Auslaß von irgeneinem/irgeneiner Speicherbehälter, Haltetank, Auffangbehälter, Wärmetauscher, Dampfturbine, Dampfmaschine, "water wall header", Entgaserauslaß und jede Kombination davon ist; und die Ergebnisse der Eisen(II)ionenbestimmung an jedem solchen Einlaß und Auslaß werden verglichen, wodurch eine Bestimmung der Korrosionsraten in einer bestimmten derartigen Kesselsystemeinheit erzielt wird.

4. Verfahren nach einem der Ansprüche 1, 2 oder 3, worin die Verfahren zum Erhalten der Kesselwasserprobe, zum Zugeben derselben zum FZ-Reagens, zum Messen der Absorption des Eisen(ll)ion/FZ-Komplexes und zum Vergleichen mit einer Standardkurve, um die in der Probe vorhandene Eisen(II)ionen-Menge zu ermitteln und zu berechnen, automatisch und kontinuierlich durchgeführt werden.

5. Verfahren nach einem der vorangegangenen Ansprüche, worin das standardisierte FZ-Reagens einen pH von 3,4 bis etwa 3,8 aufweist und zumindest 0,005 Mol pro Liter FZ-Reagens enthält.

6. Verfahren nach einem der vorangegangenen Ansprüche, worin das Volumsverhältnis zwischen Kesselwasserprobe und standardisiertem FZ-Reagens zwischen 100:1 und 1:100 liegt.

7. Verfahren nach einem der vorangegangenen Ansprüche, worin die gemessene Eisen(II)ionen-Konzentration im Bereich zwischen 0,5 und 1000 ppb (Teile pro Milliarde), bezogen auf das Gesamtkesselwasserprobenvolumen, liegt.

8. Verfahren nach einem der vorangegangenen Ansprüche, worin, nachdem die Messung der Absorption des Eisen(ll)ion/FZ-Komplexes in der Kesselwasserprobe abgeschlossen ist, eine bekannte Menge eines starken Reduktionsmittels, welches Hydroxylaminhydrochlorid, Thioglykolsäure oder deren Salze, Erythorbinsäure oder deren Salze, Hydrazin, Carbohydrazin, Natriumbisulfit oder irgendein Gemisch davon ist, der Eisen(ll)ion/FZ-Komplex-Lösung zugegeben, vermischt und ausreichend lange damit umgesetzt wird, um jede vorhandene Eisen(lll)ion-Spezies zu Eisen(II)ion zu reduzieren, welches Eisen(II)ion mit restlichem FZ-Reagens reagiert, wodurch zusätzliche Eisen(ll)ion/FZ-Komplex-Lösung gebildet wird, anschließend die Absorption dieser zusätzlichen Fe(II)-FZ-Lösung erneut gemessen, wodurch der Gesamteisengehalt gemessen wird, und dann der Eisen(III)ionen-Gehalt erhalten wird, indem der ursprüngliche Eisen(ll)ionen-Gehalt vom Gesamteisengehalt abgezogen wird.

9. Verfahren nach Anspruch 8, worin das starke Reduktionsmittel Thioglykolsäure ist.

## Revendications

1. Méthode pour déterminer les taux réels de corrosion de métaux ferreux dans des systèmes de chaudière en contact avec des eaux de chaudière contenant des produits chimiques de traitement des eaux de chaudière, laquelle méthode consiste à :
(1) Obtenir un volume connu d'un échantillon d'eau de chaudière d'une manière qui protège ledit échantillon de l'oxygène; et
(2) Ajouter ledit volume connu dudit échantillon à un volume connu d'une solution tamponnée à l'acétate à pH 2,0 à 6,0, contenant une concentration effective d'acide 3-(2-pyridyl)-5,6-diphényl-1,2,4-triazine-p,p'-disulfonique monohydraté réactif ou son sel ("FZ") et mélanger; pour ainsi former une solution d'un complexe soluble ion ferreux/FZ; et
(3) Mesurer par spectrophotométrie l'absorbance dudit complexe ion ferreux/FZ à une longueur d'onde d'environ 560 ± 30 nanomètres; et puis
(4) Calculer la quantité d'ion ferreux présent en comparant l'absorbance obtenue à l'étape (3) avec une courbe standard d'absorbance ion ferreux/FZ produite au préalable; et puis
(5) Répéter les étapes 1 à 4, si nécessaire, pour déterminer les taux réels de corrosion sur une période connue de temps.

2. Méthode selon la revendication 1 où la solution normalisée de l'acide 3-(2-pyridyl)-5,6-diphényl-1,2,4-triazine-p,p'-disulfonique réactif contient une quantité efficace dudit réactif pour former ledit complexe ion ferreux/FZ en quantités essentiellement quantitatives, même en présence desdits produits chimiques de traitement de chaudière.

3. Méthode selon la revendication 1 ou la revendication 2 où l'échantillon d'eau de chaudière est prélevé d'une unité d'un système de chaudière qui est une entrée et une sortie de condenseurs de vapeur, de l'eau d'alimentation vers la chaudière, de la purge de la chaudière ou de l'entrée et de la sortie de tout récipient de stockage, réservoir de maintien, récepteur, échangeur de chaleur, turbine, piston, collecteur de paroi d'eau, sortie de dégazeur et toute combinaison de ceux-ci; et les résultats de la détermination de l'ion ferreux à chaque entrée et sortie sont comparés, pour ainsi obtenir une détermination des taux de corrosion dans ladite unité donnée de système chaudière.

4. Méthode selon l'une quelconque des revendications 1, 2 ou 3 où les méthodes d'obtention dudit échantillon d'eau de chaudière, de son addition au réactif FZ, de la mesure de l'absorbance du complexe ion ferreux/FZ et de la comparaison avec une courbe standard pour déterminer et calculer la quantité de l'ion ferreux présent dans ledit échantillon sont accomplies automatiquement et continuellement.

5. Méthode selon l'une quelconque des revendications précédentes où le réactif FZ normalisé a un pH de 3,4 à environ 3,8 et contient au moins 0,005 mole par litre du réactif FZ.

6. Méthode selon l'une quelconque des revendications précédentes où le rapport en volume de l'échantillon d'eau de chaudière au réactif FZ normalisé est compris entre 100:1 et 1:100.

7. Méthode selon l'une quelconque des revendications précédentes où la concentration mesurée en ion ferreux est comprise entre 0,5 et 1000 parties par billion, en se basant sur le volume total de l'échantillon d'eau de chaudière.

8. Méthode selon l'une quelconque des revendications précédentes où, après la mesure de l'absorbance du complexe ion ferreux/FZ dans l'échantillon d'eau de chaudière, une quantité connue d'un agent réducteur fort qui est le chlorhydrate d'hydroxylamine, l'acide thioglycolique ou ses sels, l'acide erythorbique ou ses sels, hydrazine, le carbohydrazide, le bisulfite de sodium ou des mélanges de ceux-ci, est ajoutée à la solution du complexe ion ferreux/FZ, mélangée et on fait réagir pendant un temps suffisant pour réduire toute variété d'ion ferrique présente en ion ferreux, lequel ion ferreux réagit avec le réactif FZ résiduel pour former une solution additionnelle du complexe ion ferreux/FZ puis on remesure l'absorbance de cette solution Fe(II)-FZ additionnelle, pour ainsi mesurer la teneur totale en fer et on obtient alors la teneur en ion ferrique par soustraction de la teneur en ion ferreux d'origine de la teneur totale en fer.

9. Méthode selon la revendication 8, où l'agent réducteur fort est l'acide thioglycolique.
